# EUROPEAN PATENT APPLICATION

(11) **EP 4 043 033 A1**
(43) Date of publication of application: **17.08.2022**
(21) Application number: 20873831.0
(22) Date of filing: 08.10.2020
(51) Int. Cl.: A61K 39/395, A61K 31/56, A61K 45/00, A61P 7/04, A61P 37/06, A61P 43/00

(54) **PHARMACEUTICAL COMPOSITION WHICH CAN BE USED FOR PREVENTION AND/OR TREATMENT OF ACQUIRED HEMOPHILIA A, AND PRODUCT COMPRISING SAID PHARMACEUTICAL COMPOSITION**

(30) Priority: 11.10.2019 JP 2019188099; 28.01.2020 JP 2020011992
(71) Applicant: CHUGAI SEIYAKU KABUSHIKI KAISHA, Tokyo 115-8543 (JP)
(72) Inventor: YONEYAMA, Koichiro, Tokyo 103-8324 (JP); NAGAMI, Sayaka, Tokyo 103-8324 (JP)
(74) Representative: Vossius & Partner Patentanwälte Rechtsanwälte mbB
(86) International application number: PCT/JP2020/038069
(87) International publication number: WO 2021/070885

(57) **Abstract**

The present inventors discovered that administration of a pharmaceutical composition comprising emicizumab according to a predetermined administration regimen has the potential to effectively prevent and/or treat acquired hemophilia A.

## Description

### [Technical Field]

The present disclosure relates to pharmaceutical compositions used in the prevention and/or treatment of acquired hemophilia A in novel administration regimens, and products comprising the pharmaceutical compositions. More specifically, the present disclosure relates to pharmaceutical compositions comprising emicizumab characterized by being administered for at least two consecutive days (daily loading administration), and products comprising such a pharmaceutical composition and a document concerning its administration.

### [Background Art]

Hemophilia is a hemorrhagic disease caused by a congenital deficiency or dysfunction of coagulation factor VIII (FVIII) or coagulation factor IX (FIX). The former is called hemophilia A and the latter is called hemophilia B.

For bleeding in hemophilia A patients, FVIII formulations are generally administered on demand (on-demand therapy). In recent years, FVIII formulations are also administered prophylactically to prevent bleeding events (regular replacement therapy; NPLs 1 and 2). The half-life of FVIII formulations in blood is approximately 8 to 19 hours. Therefore, for continuous prevention, FVIII formulations are administered to patients one to three times a week (NPLs 3 and 4). In on-demand therapy, FVIII formulations are also additionally administered at regular intervals as necessary to prevent reoccurrence of bleeding. In addition, FVIII formulations are mainly administered at home, but since they are administered intravenously, the difficulty of securing a blood vessel is a problem. Therefore, there has been a strong need for pharmaceutical agents with a lesser burden regarding their administration as compared to FVIII formulations.

Occasionally, repeated replacement therapy results in development of antibodies against FVIII (inhibitors) in hemophilia A patients. Such inhibitors counteract the effects of the FVIII formulations. For bleeding in patients who have developed inhibitors (inhibitor patients), bypassing agents are administered. Their mechanisms of action are not dependent on FVIII function, that is, on the function of catalyzing the activation of blood coagulation factor X (FX) by activated blood coagulation factor IX (FIXa). Therefore, in some cases, bypassing agents cannot sufficiently stop the bleeding. Recently, results suggesting the effectiveness of regular administration therapy of bypassing agents have been obtained, but this has not yielded a sufficient effect to suppress bleeding as compared to FVIII formulations. Accordingly, there has been a strong need for therapeutic agents that can be administered subcutaneously, as well as long-acting therapeutic agents that can be administered less frequently, regardless of the presence of inhibitors.

Recently, as a means for solving the problem, a bispecific antibody that functionally substitutes for FVIII, emicizumab (trade name: Hemlibra, ACE910, RO5534262) and its use were disclosed (PTLs 1, 2, 3, 4, and 5, NPLs 5, 6, 7, and 8).

Emicizumab is a recombinant humanized bispecific antibody that binds to (a) FIX and/or FIXa and (b) FX and/or activated blood coagulation factor FX (FXa), and substitutes for the cofactor function of FVIII. Emicizumab has recently been approved in the United States and Japan as a recombinant drug that suppresses the bleeding tendency in patients with congenital FVIII deficiency. This product is a long-acting subcutaneous preparation that allows low frequency administration-subcutaneous administration at 3 mg/kg (body weight) per time, four times at weekly intervals, and thereafter, at any of the dosage and administration regimens of 1.5 mg/kg (body weight) per time at weekly intervals, 3 mg/kg (body weight) per time at 2-week intervals, or 6 mg/kg (body weight) per time at 4-week intervals. Its efficacy has been confirmed irrespective of the presence or absence of FVIII inhibitors.

On the other hand, unlike the above case in patients with congenital hemophilia A where an alloantibody is generated due to replacement therapy and acts as an inhibitor, also known is acquired hemophilia A, which is an autoimmune disease in which an autoantibody against FVIII appears acquiredly and acts as an inhibitor (NPL 11).

It has been reported that 70-80% of patients with acquired hemophilia A usually achieve complete remission (CR) by treatment with various immunosuppressive therapies (hereinafter, "immunotherapy"). However, the median time from the start of treatment to the achievement of CR is several months, and the median time to achievement of partial remission (PR) is also about a month, during which time, the patients are exposed to the risk of bleeding and susceptibility to infections as a result of the immunotherapy. In fact, many of the causes of death in acquired hemophilia A are serious bleeding and severe infections. Therefore, when immunotherapy is performed, treatment with a drug that complements the function of FVIII is required until the effect is obtained. In addition, for patients for whom immunotherapy cannot be used or who have not obtained a significant effect even after immunotherapy, continuous administration of a drug that complements the function of FVIII is required. Since patients with acquired hemophilia A cannot be expected to have a therapeutic effect with FVIII preparations due to the inhibitor produced, bypassing agents are used, which are not sufficiently effective in suppressing bleeding as compared to FVIII preparations. Therefore, it is hoped that emicizumab, which is effective even in the presence of an inhibitor, will be applied to acquired hemophilia A.

In general, many of the bleeding symptoms of acquired hemophilia A are more serious than those of patients with congenital hemophilia, and the risk of bleeding to death is high. In addition, since the risk of bleeding in the early stage of onset is high and this risk is gradually reduced by immunotherapy, when emicizumab is used as a therapeutic agent for acquired hemophilia A, it is necessary that an effective plasma emicizumab concentration is attained at an early stage after the start of administration and that this effective concentration is maintained.

This requirement applies regardless of whether emicizumab administration is performed under immunotherapy. Under immunotherapy, the effect is regularly evaluated by measuring, for example, FVIII activity and inhibitor titer of the patient, and if an effect is observed, the dose of the immunotherapeutic agent is reduced or discontinued in a timely manner, and if not, the agent is changed or an additional one is added. When emicizumab is administered in parallel to the administration of an immunotherapeutic agent, it is expected that the continuation/discontinuation of emicizumab administration would also be decided based on the result of this determination of effect. Therefore, it is desirable to apply such a monitoring and decision-making system for continuation/discontinuation of treatment at an early stage along with the use of emicizumab.

Regarding the use of emicizumab for acquired hemophilia A, research reports confirming the hemostatic effect of emicizumab in an acquired hemophilia A model experiment (NPL 7 and 12), and reports of emicizumab administration in acquired hemophilia A patients (NPL 13 and 14) are known. However, these reports do not describe the problem and solution for shortening the time until an emicizumab therapeutic effect is obtained in acquired hemophilia A.

### [Citation List]

### [Patent Literature]

[PTL 1] WO 2005/035754
[PTL 2] WO 2005/035756
[PTL 3] WO 2006/109592
[PTL 4] WO 2012/067176
[PTL 5] WO 2015/194233

### [Non-Patent Literature]

[NPL 1] Blood 58, 1-13 (1981)
[NPL 2] Nature 312, 330-337 (1984)
[NPL 3] Nature 312, 337-342 (1984)
[NPL 4] Biochim.Biophys.Acta 871, 268-278 (1986)
[NPL 5] Nature Medicine 18, 1570-1574(2012)
[NPL 6] PLOS ONE 8, 1-13(2013)
[NPL 7] J Thromb Haemost. 12, 206-213(2014)
[NPL 8] Blood 127(13), 1633-1641(2016)
[NPL 9] N Engl J Med. 374(21), 2044-2053(2016)
[NPL 10] XXV Congress of the International Society on Thrombosis and Haemostasis, Toronto, Canada, June 20-25, 2015. Abstr AS017.
[NPL 11] Japanese Journal of Thrombosis and Hemostasis 28(6), 715-747 (2017)
[NPL 12] Blood 124(20), 3165-3171 (2014)
[NPL 13] Am J Case Rep. 20, 1046-1048 (2019)
[NPL 14] Res Pract Thromb Haemost. 3(3), 420-423 (2019)

### [Summary of Invention]

### [Technical Problem]

This disclosure was made in view of such circumstances, and one of the objectives is to provide optimal emicizumab administration regimens for the treatment of acquired hemophilia A.

### [Solution to Problem]

The inventors of the present disclosure conducted diligent research to achieve the above objective and succeeded in discovering more effective administration regimens of pharmaceutical compositions comprising emicizumab for the prevention and/or treatment of acquired hemophilia A. More specifically, the present inventors discovered that by administering emicizumab for at least two consecutive days (daily loading administration), an effective plasma emicizumab concentration can be obtained at an early stage after the start of administration, and that after approximately one week and thereafter following the initial administration, the effective concentration can be maintained by administering emicizumab at 1-to 4-week intervals (1- to 4-week interval maintenance administration).

The present disclosure is based on such findings, and specifically includes the embodiments exemplified below.
[1] A pharmaceutical composition for use in treating acquired hemophilia A and/or reducing the incidence of acquired hemophilia A, wherein the composition comprises emicizumab, wherein emicizumab is administered at an antibody dose of 6 mg/kg on Day 1, at an antibody dose of 3 to 6 mg/kg on Day 2, and at an antibody dose of 1.5 to 3 mg/kg weekly from Day 8, wherein the weekly administration from Day 8 is repeated one or more times.
[2] The pharmaceutical composition of [1], wherein the administration on Day 1, the administration on Day 2, and the weekly administration from Day 8 are each performed in a single dose.
[3] The pharmaceutical composition of [1] or [2], wherein the administration on Day 1, the administration on Day 2, and the weekly administration from Day 8 are each performed subcutaneously.
[4] The pharmaceutical composition of any one of [1] to [3], wherein emicizumab is administered at an antibody dose of 3 mg/kg on Day 2.
[5] The pharmaceutical composition of any one of [1] to [4], wherein emicizumab is administered at an antibody dose of 1.5 to 2 mg/kg weekly from Day 8, wherein the weekly administration from Day 8 is repeated one or more times.
[6] The pharmaceutical composition of any one of [1] to [5], wherein emicizumab is administered at an antibody dose of 1.5 mg/kg weekly from Day 8, wherein the weekly administration from Day 8 is repeated one or more times.
[7] The pharmaceutical composition of any one of [1] to [6], which is used under administration of an immunosuppressive drug.
[8] The pharmaceutical composition of [7], wherein the immunosuppressive drug is a steroid drug.
[9] A product comprising (i) a container; (ii) a pharmaceutical composition comprising emicizumab in the container, and (iii) a document instructing that emicizumab be administered at an antibody dose of 6 mg/kg on Day 1, at an antibody dose of 3 to 6 mg/kg on Day 2, and at an antibody dose of 1.5 to 3 mg/kg weekly from Day 8, and that the weekly administration from Day 8 be repeated one or more times.
[10] The product of [9], wherein the administration on Day 1, administration on Day 2, and weekly administration from Day 8 are each performed in a single dose.
[11] The product of [9] or [10], wherein the administration on Day 1, administration on Day 2, and weekly administration from Day 8 are each performed subcutaneously.
[12] The product of any one of [9] to [11], wherein emicizumab is administered at an antibody dose of 3 mg/kg on Day 2.
[13] The product of any one of [9] to [12], wherein emicizumab is administered at an antibody dose of 1.5 mg/kg weekly from Day 8, wherein the weekly administration from Day 8 is repeated one or more times.
[14] The product of any one of [9] to [13], which comprises said document instructing that emicizumab be used in combination with an immunosuppressive drug.
[15] A pharmaceutical composition for use in treating acquired hemophilia A and/or reducing the incidence of acquired hemophilia A, which comprises emicizumab, wherein emicizumab is administered at an antibody dose of 6 mg/kg on Day 1, at an antibody dose of 3 to 6 mg/kg on Day 2, and at an antibody dose of 3 to 4 mg/kg every 2 weeks from Day 8, or at an antibody dose of 6 mg/kg every 4 weeks from Day 8, wherein the administration every 2 weeks or every 4 weeks from Day 8 is repeated one or more times.
[16] The pharmaceutical composition of [15], wherein emicizumab is administered at an antibody dose of 3 mg/kg every 2 weeks from Day 8, wherein the administration every 2 weeks from Day 8 is repeated one or more times.
[17] The pharmaceutical composition of [15] or [16], wherein the administration on Day 1, the administration on Day 2, and the administration every 2 weeks or every 4 weeks from Day 8 are each performed in a single dose.
[18] The pharmaceutical composition of any one of [15] to [17], wherein the administration on Day 1, the administration on Day 2, and the administration every 2 weeks or every 4 weeks from Day 8 are each performed subcutaneously.
[19] The pharmaceutical composition of any one of [15] to [18], wherein emicizumab is administered at an antibody dose of 3 mg/kg on Day 2.
[20] The pharmaceutical composition of any one of [15] to [19], which is used under administration of an immunosuppressive drug.
[21] The pharmaceutical composition of [20], wherein the immunosuppressive drug is a steroid drug.
[22] A pharmaceutical composition for use in treating acquired hemophilia A and/or reducing the incidence of acquired hemophilia A, which comprises emicizumab, wherein emicizumab is administered at an antibody dose of 6 mg/kg on Day 1, at an antibody dose of 3 to 6 mg/kg on Day 2, at an antibody dose of 1.5 to 3 mg/kg on Day 8, and at an antibody dose of 3 to 4 mg/kg every 2 weeks from Day 15, or at an antibody dose of 6 mg/kg every 4 weeks from Day 15, wherein the administration every 2 weeks or every 4 weeks from Day 15 is repeated one or more times.
[23] The pharmaceutical composition of [22], wherein emicizumab is administered at an antibody dose of 3 mg/kg every 2 weeks from Day 15, wherein the administration every 2 weeks from Day 15 is repeated one or more times.
[24] The pharmaceutical composition of [22] or [23], wherein the administration on Day 1, the administration on Day 2, the administration on Day 8, and the administration every 2 weeks or every 4 weeks from Day 15 are each performed in a single dose.
[25] The pharmaceutical composition of any one of [22] to [24], wherein the administration on Day 1, the administration on Day 2, the administration on Day 8, and the administration every 2 weeks or every 4 weeks from Day 15 are each performed subcutaneously.
[26] The pharmaceutical composition of any one of [22] to [25], wherein emicizumab is administered at an antibody dose of 3 mg/kg on Day 2.
[27] The pharmaceutical composition of any one of [22] to [26], wherein emicizumab is administered at an antibody dose of 1.5 mg/kg on Day 8.
[28] The pharmaceutical composition of any one of [22] to [27], which is used under administration of an immunosuppressive drug.
[29] The pharmaceutical composition of [28], wherein the immunosuppressive drug is a steroid drug.

Further, the present disclosure includes aspects exemplified below.

[30] A pharmaceutical composition for use in treating acquired hemophilia A and/or reducing the incidence of acquired hemophilia A, which comprises emicizumab, wherein emicizumab is administered at an antibody dose of 6 mg/kg on Day 1, at an antibody dose of 3 to 6 mg/kg on Day 2, at an antibody dose of 1.5 to 6 mg/kg on Day 3, and at an antibody dose of 1.5 to 3 mg/kg every week from Day 8, at an antibody dose of 3 mg/kg every 2 weeks from Day 8, or at an antibody dose of 6 mg/kg every 4 weeks from Day 8, wherein the administration every week, every 2 weeks, or every 4 weeks from Day 8 is repeated one or more times.

[31] The pharmaceutical composition of [30], wherein the administration on Day 1, the administration on Day 2, the administration on Day 3, and the administration every week, every 2 weeks, or every 4 weeks, from Day 8 are each performed in a single dose.

[32] The pharmaceutical composition of [30], wherein the administration on Day 1, the administration on Day 2, the administration on Day 3, and the administration every week, every 2 weeks, or every 4 weeks, from Day 8 are each performed subcutaneously.

[33] The pharmaceutical composition of any one of [30] to [32], wherein emicizumab is administered at an antibody dose of 3 mg/kg on Day 2.

[34] The pharmaceutical composition of any one of [30] to [33], wherein emicizumab is administered at an antibody dose of 1.5 mg/kg on Day 3.

[35] The pharmaceutical composition of any one of [30] to [34], wherein emicizumab is administered at an antibody dose of 1.5 mg/kg every week from Day 8, wherein the administration every week from Day 8 is repeated one or more times.

[36] The pharmaceutical composition of any one of [30] to [35], which is used under administration of an immunosuppressive drug.

[37] The pharmaceutical composition of [36], wherein the immunosuppressive drug is a steroid drug.

[38] A method of treating acquired hemophilia A and/or reducing the incidence of acquired hemophilia A, wherein the method comprises administering emicizumab at an antibody dose of 6 mg/kg on Day 1, at an antibody dose of 3 to 6 mg/kg on Day 2, and at an antibody dose of 1.5 to 3 mg/kg every week from Day 8, wherein the administration every week from Day 8 is repeated one or more times.

[39] The method of [38], wherein the administration on Day 1, the administration on Day 2, and the administration every week from Day 8 are each performed in a single dose.

[40] The method of [38] or [39], wherein the administration on Day 1, the administration on Day 2, and the administration every week from Day 8 are each performed subcutaneously.

[41] The method of any one of [38] to [40], wherein emicizumab is administered at an antibody dose of 3 mg/kg on Day 2.

[42] The method of any one of [38] to [41], wherein emicizumab is administered at an antibody dose of 1.5 to 2 mg/kg every week from Day 8, wherein the administration every week from Day 8 is repeated one or more times.

[43] The method of any one of [38] to [42], wherein emicizumab is administered at an antibody dose of 1.5 mg/kg every week from Day 8, wherein the administration every week from Day 8 is repeated one or more times.

[44] The method of any one of [38] to [43], which is used under administration of an immunosuppressive drug.

[45] The method of [44], wherein the immunosuppressive drug is a steroid drug.

[46] Use of emicizumab for the production of a therapeutic agent for acquired hemophilia A and/or an agent for reducing the incidence of acquired hemophilia A, wherein emicizumab is administered at an antibody dose of 6 mg/kg on Day 1, at an antibody dose of 3 to 6 mg/kg on Day 2, and at an antibody dose of 1.5 to 3 mg/kg every week from Day 8, wherein the administration every week from Day 8 is repeated one or more times.

[47] The use of [46], wherein the administration on Day 1, the administration on Day 2, and the administration every week from Day 8 are each performed in a single dose.

[48] The use of [46] or [47], wherein the administration on Day 1, the administration on Day 2, and the administration every week from Day 8 are each performed subcutaneously.

[49] The use of any one of [46] to [48], wherein emicizumab is administered at an antibody dose of 3 mg/kg on Day 2.

[50] The use of any one of [46] to [49], wherein emicizumab is administered at an antibody dose of 1.5 to 2 mg/kg every week from Day 8, wherein the administration every week from Day 8 is repeated one or more times.

[51] The use of any one of [46] to [50], wherein emicizumab is administered at an antibody dose of 1.5 mg/kg every week from Day 8, wherein the administration every week from Day 8 is repeated one or more times.

[52] The use of any one of [46] to [51], which is used under administration of an immunosuppressive drug.

[53] The use of [52], wherein the immunosuppressive drug is a steroid drug.

[54] Emicizumab for use in treatment of acquired hemophilia A and/or in reduction of the incidence of acquired hemophilia A, which is administered at an antibody dose of 6 mg/kg on Day 1, at an antibody dose of 3 to 6 mg/kg on Day 2, and at an antibody dose of 1.5 to 3 mg/kg every week from Day 8, wherein the administration every week from Day 8 is repeated one or more times.

[55] The emicizumab of [54], wherein the administration on Day 1, the administration on Day 2, and the administration every week from Day 8 are each performed in a single dose.

[56] The emicizumab of [54] or [55], wherein the administration on Day 1, the administration on Day 2, and the administration every week from Day 8 are each performed subcutaneously.

[57] The emicizumab of any one of [54] to [56], which is administered at an antibody dose of 3 mg/kg on Day 2.

[58] The emicizumab of any one of [54] to [57], which is administered at an antibody dose of 1.5 to 2 mg/kg every week from Day 8, wherein the administration every week from Day 8 is repeated one or more times.

[59] The emicizumab of any one of [54] to [58], which is administered at an antibody dose of 1.5 mg/kg every week from Day 8, wherein the administration every week from Day 8 is repeated one or more times.

[60] The emicizumab of any one of [54] to [59], which is used under administration of an immunosuppressive drug.

[61] The emicizumab of [60], wherein the immunosuppressive drug is a steroid drug.

[62] A method of treating acquired hemophilia A and/or reducing the incidence of acquired hemophilia A, wherein the method comprises administering emicizumab at an antibody dose of 6 mg/kg on Day 1, at an antibody dose of 3 to 6 mg/kg on Day 2, and at an antibody dose of 3 to 4 mg/kg every 2 weeks from Day 8 or at an antibody dose of 6 mg/kg every 4 weeks from Day 8, wherein the administration every 2 weeks or every 4 weeks from Day 8 is repeated one or more times.

[63] A method of treating acquired hemophilia A and/or reducing the incidence of acquired hemophilia A, wherein the method comprises administering emicizumab at an antibody dose of 6 mg/kg on Day 1, at an antibody dose of 3 to 6 mg/kg on Day 2, at an antibody dose of 1.5 to 3 mg/kg on Day 8, and at an antibody dose of 3 to 4 mg/kg every 2 weeks from Day 15 or at an antibody dose of 6 mg/kg every 4 weeks from Day 15, wherein the administration every 2 weeks or every 4 weeks from Day 15 is repeated one or more times.

[64] A method of treating acquired hemophilia A and/or reducing the incidence of acquired hemophilia A, wherein the method comprises administering emicizumab at an antibody dose of 6 mg/kg on Day 1, at an antibody dose of 3 to 6 mg/kg on Day 2, at an antibody dose of 1.5 to 6 mg/kg on Day 3, and at an antibody dose of 1.5 to 3 mg/kg every week from Day 8, at an antibody dose of 3 to 4 mg/kg every 2 weeks from Day 8, or at an antibody dose of 6 mg/kg every 4 weeks from Day 8, wherein the administration every week, every 2 weeks, or every 4 weeks from Day 8 is repeated one or more times.

[65] Use of emicizumab for the production of a therapeutic agent for acquired hemophilia A and/or an agent for reducing the incidence of acquired hemophilia A, wherein emicizumab is administered at an antibody dose of 6 mg/kg on Day 1, at an antibody dose of 3 to 6 mg/kg on Day 2, and at an antibody dose of 3 to 4 mg/kg every 2 weeks from Day 8 or at an antibody dose of 6 mg/kg every 4 weeks from Day 8, wherein the administration every 2 weeks or every 4 weeks from Day 8 is repeated one or more times.

[66] Use of emicizumab for the production of a therapeutic agent for acquired hemophilia A and/or an agent for reducing the incidence of acquired hemophilia A, wherein emicizumab is administered at an antibody dose of 6 mg/kg on Day 1, at an antibody dose of 3 to 6 mg/kg on Day 2, at an antibody dose of 1.5 to 3 mg/kg on Day 8, and at an antibody dose of 3 to 4 mg/kg every 2 weeks from Day 15 or at an antibody dose of 6 mg/kg every 4 weeks from Day 15, wherein the administration every 2 weeks or every 4 weeks from Day 15 is repeated one or more times.

[67] Use of emicizumab for the production of a therapeutic agent for acquired hemophilia A and/or an agent for reducing the incidence of acquired hemophilia A, wherein emicizumab is administered at an antibody dose of 6 mg/kg on Day 1, at an antibody dose of 3 to 6 mg/kg on Day 2, at an antibody dose of 1.5 to 6 mg/kg on Day 3, and at an antibody dose of 1.5 to 3 mg/kg every week from Day 8, at an antibody dose of 3 to 4 mg/kg every 2 weeks from Day 8, or at an antibody dose of 6 mg/kg every 4 weeks from Day 8, wherein the administration every week, every 2 weeks, or every 4 weeks from Day 8 is repeated one or more times.

[68] Emicizumab for use in treatment of acquired hemophilia A and/or in reduction of the incidence of acquired hemophilia A, wherein emicizumab is administered at an antibody dose of 6 mg/kg on Day 1, at an antibody dose of 3 to 6 mg/kg on Day 2, and at an antibody dose of 3 to 4 mg/kg every 2 weeks from Day 8 or at an antibody dose of 6 mg/kg every 4 weeks from Day 8, wherein the administration every 2 weeks or every 4 weeks from Day 8 is repeated one or more times.

[69] Emicizumab for use in treatment of acquired hemophilia A and/or in reduction of the incidence of acquired hemophilia A, wherein emicizumab is administered at an antibody dose of 6 mg/kg on Day 1, at an antibody dose of 3 to 6 mg/kg on Day 2, at an antibody dose of 1.5 to 3 mg/kg on Day 8, and at an antibody dose of 3 to 4 mg/kg every 2 weeks from Day 15 or at an antibody dose of 6 mg/kg every 4 weeks from Day 15, wherein the administration every 2 weeks or every 4 weeks from Day 15 is repeated one or more times.

[70] Emicizumab for use in treatment of acquired hemophilia A and/or in reduction of the incidence of acquired hemophilia A, wherein emicizumab is administered at an antibody dose of 6 mg/kg on Day 1, at an antibody dose of 3 to 6 mg/kg on Day 2, at an antibody dose of 1.5 to 6 mg/kg on Day 3, and at an antibody dose of 1.5 to 3 mg/kg every week from Day 8, at an antibody dose of 3 to 4 mg/kg every 2 weeks from Day 8, or at an antibody dose of 6 mg/kg every 4 weeks from Day 8, wherein the administration every week, every 2 weeks, or every 4 weeks from Day 8 is repeated one or more times.

### [Brief Description of Drawings]

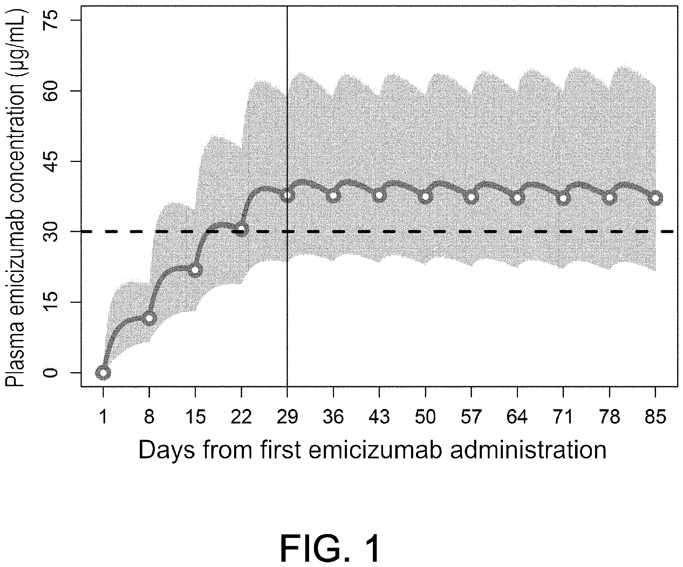

Fig. 1 shows the simulated time course of plasma emicizumab concentration over time (approved 1-week regimen). The X-axis shows the number of days elapsed when the first day of administration of emicizumab is taken as Day 1 (1st day). The Y-axis indicates plasma emicizumab concentration (µg/mL). The circles and solid line show the time course of the predicted median with the trough levels plotted, the peripheral area shows the 5th to 95th percentile range, and the vertical solid line shows Day 29, which is expected to be the last trough time point before PR achievement in approximately half of the patients, and the horizontal broken line is the estimated effective concentration of 30 µg/mL. The dosage and administration comprised repeated subcutaneous administration of 3 mg/kg for 4 weeks at 1-week intervals (loading administration) followed by repeated subcutaneous administration of 1.5 mg/kg at 1-week intervals thereafter (maintenance administration).

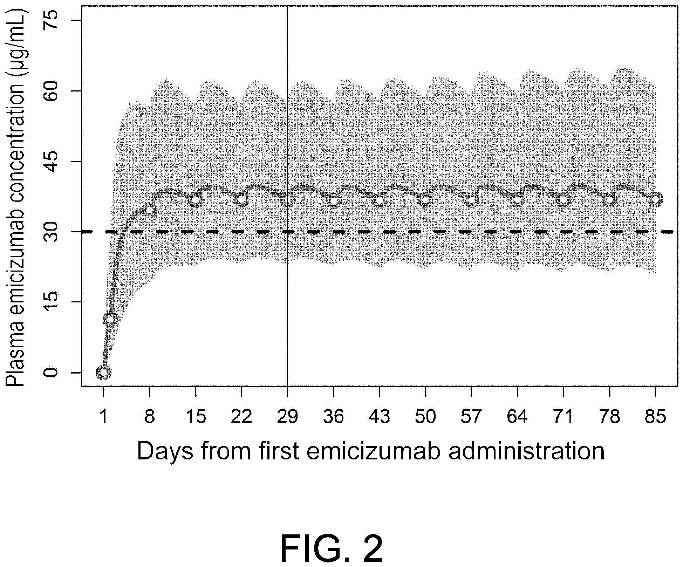

Fig. 2 shows the simulated time course of plasma emicizumab concentration over time (daily loading + 1-week interval maintenance administration regimen). The X-axis shows the number of days elapsed when the first day of administration of emicizumab is taken as Day 1 (1st day). The Y-axis indicates plasma emicizumab concentration (µg/mL). The circles and solid line show the time course of the predicted median with the trough levels plotted, the peripheral area shows the 5th to 95th percentile range, and the vertical solid line shows Day 29, which is expected to be the last trough time point before PR achievement in approximately half of the patients, and the horizontal broken line is the estimated effective concentration of 30 µg/mL. The dosage and administration comprised subcutaneous administration of 6 mg/kg and 3 mg/kg on Day 1 and Day 2, respectively (loading administration), followed by repeated subcutaneous administration of 1.5 mg/kg from Day 8 at weekly intervals (maintenance administration).

### [Description of Embodiments]

Emicizumab (ACE910, RO5534262) is a bispecific antigen-binding molecule that recognizes (a) blood coagulation factor IX (FIX) and/or activated blood coagulation factor IX (FIXa) and (b) blood coagulation factor X (FX) and/or activated blood coagulation factor X (FXa), and has an activity of functionally substituting for coagulation factor VIII (FVIII).

In the present invention, the phrase "functionally substitute for FVIII" means that (a) FIX and/or FIXa, and (b) FX and/or FXa are recognized, and the activation of FX by FIXa is promoted (FXa generation by FIXa is promoted). FXa generation-promoting activity can be evaluated using, for example, a measurement system comprising FIXa, FX, the synthetic substrate S-2222 (a synthetic substrate of FXa), and phospholipids. Such a measurement system shows the correlation between the severity of the disease and the clinical symptoms in hemophilia A cases (Rosen S, Andersson M, Blomback M et al. Clinical applications of a chromogenic substrate method for determination of FVIII activity. Thromb Haemost 1985; 54: 811-23).

Such antigen-binding molecules (such as antibodies) recognizing (a) FIX and/or FIXa and (b) FX and/or FXa can be obtained according to methods described, for example, in WO2005/035756, WO2006/109592, and WO2012/067176. Specifically, based on the sequences of antibodies against FIX and/or FIXa and antibodies against FX and/or FXa, antibodies can be generated using genetic recombination techniques known to those skilled in the art. Polynucleotide(s) encoding an antibody can be constructed based on the sequences of the antibodies against FIX and/or FIXa and antibodies against FX and/or FXa, and this can be inserted into an expression vector and subsequently expressed in appropriate host cells (see for example, Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; and Bird, R. E. and Walker, B. W., Trends Biotechnol. (1991) 9, 132-137).

In the present invention, the phrases "functionally substitute for FVIII" and "functionally substitute for FVIIIa" are used interchangeably.

Emicizumab is a bispecific antibody in which a first polypeptide and a third polypeptide are associated and a second polypeptide and a fourth polypeptide are associated, and has the following structure:
(a) a bispecific antibody comprising a first polypeptide which is an H chain containing an H chain variable region containing CDR 1, 2, and 3 amino acid sequences of SEQ ID NOs: 1, 2, and 3, respectively; a second polypeptide which is an H chain containing an H chain variable region containing CDR 1, 2, and 3 amino acid sequences of SEQ ID NOs: 6, 7, and 8, respectively; and a third and fourth polypeptide which are a commonly shared L chain containing an L chain variable region containing CDR 1, 2, and 3 amino acid sequences of SEQ ID NOs: 11, 12, and 13, respectively;
(b) a bispecific antibody comprising a first polypeptide which is an H chain containing an H chain variable region amino acid sequence of SEQ ID NO: 4;
   a second polypeptide which is an H chain containing an H chain variable region amino acid sequence of SEQ ID NO: 9; and a third and fourth polypeptide which are a commonly shared L chain containing an L chain variable region amino acid sequence of SEQ ID NO: 14; or
(c) a bispecific antibody comprising a first polypeptide which is an H chain containing the amino acid sequence of SEQ ID NO: 5; a second polypeptide which is an H chain containing the amino acid sequence of SEQ ID NO: 10; and a third and fourth polypeptide which are a commonly shared L chain containing the amino acid sequence of SEQ ID NO: 15 (Q499-z121/J327-z119/L404-k).

Pharmaceutical compositions of the present invention which are used for therapeutic or preventive purposes can be prepared by mixing emicizumab, if necessary, with suitable pharmaceutically acceptable carriers, vehicles, and such and made into a freeze-dry formulation or a solution formulation.

Examples of suitable pharmaceutically acceptable carriers and vehicles include sterilized water, physiological saline, stabilizers, excipients, antioxidants (such as ascorbic acid), buffers (such as phosphate, citrate, histidine, and other organic acids), antiseptics, surfactants (such as PEG and Tween), chelating agents (such as EDTA), and binders. They may also contain other low-molecular-weight polypeptides, proteins such as serum albumin, gelatin, and immunoglobulins, amino acids such as glycine, glutamine, asparagine, glutamic acid, aspartic acid, methionine, arginine, and lysine, sugars and carbohydrates such as polysaccharides and monosaccharides, and sugar alcohols such as mannitol and sorbitol. When preparing an aqueous solution for injection, for example, physiological saline and isotonic solutions containing glucose and other adjuvants such as D-sorbitol, D-mannose, D-mannitol, and sodium chloride may be used, and appropriate solubilizers such as alcohol (for example, ethanol), polyalcohols (such as propylene glycol and PEG), and nonionic surfactants (such as polysorbate 80, polysorbate 20, poloxamer 188, and HCO-50) may be used in combination. By mixing hyaluronidase into the formulation, a larger fluid volume can be administered subcutaneously (Expert Opin Drug Deliv. 2007 Jul; 4(4): 427-40). Further, the pharmaceutical compositions of the present invention may be preliminarily loaded in a syringe. Meanwhile, the solution formulation can be prepared according to the method described in WO 2011/090088.

If necessary, emicizumab can be encapsulated in microcapsules (e.g., those made of hydroxymethylcellulose, gelatin, and poly(methylmetacrylate)), or prepared as colloidal drug delivery systems (e.g., liposomes, albumin microspheres, microemulsion, nanoparticles, and nanocapsules) (see, for example, "Remington's Pharmaceutical Science 16th edition", Oslo Ed. (1980)). Methods for preparing the pharmaceutical agents as controlled-release pharmaceutical agents are also well known, and such methods may be applied to emicizumab (Langer et al., J. Biomed. Mater. Res. 15: 267-277 (1981); Langer, Chemtech. 12: 98-105 (1982); U.S. Patent No. 3,773,919; European Patent Application Publication No. EP 58,481; Sidman et al., Biopolymers 22: 547-556 (1983); EP 133,988).

A preferable liquid formulation is as follows.
20 mg/ml to 180 mg/ml emicizumab,
0.2 mg/ml to 1 mg/ml of poloxamer 188,
10 mM to 40 mM of histidine-aspartic acid buffer,
100 mM to 300 mM of arginine, at a pH of about 4.5 to 6.5.
In addition, the dose of emicizumab in one vial can be 30 mg, 60 mg, 90 mg, 105 mg or 150 mg.

"Administration" as used herein refers to administration carried out in a single dose or multiple doses. Administration may be via any appropriate route, for example, intravenously by bolus injection or continuous infusion for a given period, intramuscularly, intraperitoneally, intracerebrospinally, transdermally, subcutaneously, intraarticularly, sublingually, intrasynovially, orally, by inhalation, locally, or externally. Intravenous administration (IV) or subcutaneous administration (SC) is preferred.

Herein, the dose of emicizumab is expressed by the antibody dose, for example, "6 mg/kg (body weight)".

"Administration on Day 1" refers to the first administration of emicizumab to a patient for the prevention and/or treatment of acquired hemophilia A. Further, "administration on Day 2" refers to the day following the administration on Day 1 which is counted as the 1st day. "Administration on Day 8" refers to the 8th day after the administration on Day 1 which is counted as the 1st day. The same applies to other dates.

The term "every week" as used herein can be rephrased as "weekly" or "at 1-week intervals". The same applies to 2 weeks, 4 weeks, and so on. In addition, "4 weeks" is used interchangeably with "one month".

Herein, "administration is repeated one or more times" means that administration is repeated once or multiple times. An "administration interval" (interval between individual administrations) refers to the interval between the nth dose (n is an integer of 1 or more) and the (*n*+1)th dose.

Emicizumab has been approved as a low-frequency subcutaneous preparation that is repeatedly administered subcutaneously at 3 mg/kg (body weight) at 1-week intervals for 4 weeks (1-week interval loading administration), followed by repeated subcutaneous administration of 1.5 mg/kg at 1-week intervals, 3 mg/kg at 2-week intervals, or 6 mg/kg at 4-week intervals (1- to 4-week interval maintenance administration) (approved 1-week, 2-week or 4-week interval regimens).

On the other hand, in the administration regimen of the present disclosure (daily loading + 1- to 4-week interval maintenance administration regimen), emicizumab is administered on at least 2 consecutive days (daily loading administration) so as to obtain an effective emicizumab concentration in plasma at an early stage after the start of administration, and this effective concentration can be maintained by administering emicizumab at intervals of 1 to 4 weeks (1- to 4-week interval maintenance administration) from about one week after the initial administration.

Herein, "loading" administration generally refers to administration to a patient performed at an early stage after the start of administration, and is followed by maintenance administration at various doses and administration intervals. In one aspect, the loading administration is carried out zero to 24 times, preferably at least once, at least twice, at least three times, at least four times, or more, and preferably twice or three times. Usually, loading administration is performed at intervals of several days, such as about 2 to 6 days or 1 to 4 weeks, for example, approximately every week, approximately every 2 weeks, approximately every 3 weeks, or approximately every 4 weeks (every month). However, the administration regimen of the present disclosure (daily loading + 1- to 4-week interval maintenance administration regimen) is characterized in that the loading administration is carried out daily, that is, the administration interval of the loading administration is one day. In one aspect, the loading administration is performed at a dose of 1.5 mg/kg to 6 mg/kg, preferably 3 mg/kg or 6 mg/kg of the antibody. Loading administration is to allow the plasma concentration of a therapeutic agent to reach its effective concentration range as early as possible and become stable (reach the steady state) as early as possible.

In certain embodiments, an antibody dose of 6 mg/kg is subcutaneously administered once on Day 1, then an antibody dose of 3 to 6 mg/kg is subcutaneously administered once on Day 2, and optionally, an additional antibody dose of 1.5 to 6 mg is administered subcutaneously once on Day 3.

Herein, "maintenance" administration (continued administration) refers to administration to a patient that is performed over a treatment period after the plasma concentration of a therapeutic agent has reached its effective concentration range as a result of loading administration. Maintenance administration can be performed with different maintenance doses and different administration intervals in combination.

In one aspect, maintenance administration is done at an antibody (emicizumab) dose of 1.5 to 6 mg/kg (body weight), e.g., at an antibody dose of 1.5 mg/kg, 1.75 mg/kg, 1.8 mg/kg, 2 mg/kg, 2.1 mg/kg, 2.25 mg/kg, 2.4 mg/kg, 2.5 mg/kg, 2.7 mg/kg, 2.75 mg/kg, 3 mg/kg, 3.5 mg/kg, 3.6 mg/kg, 4 mg/kg, 4.2 mg/kg, 4.5 mg/kg, 4.8 mg/kg, 5 mg/kg, 5.4 mg/kg, 5.5 mg/kg, or 6 mg/kg. In one aspect, the maintenance administration interval is 1 to 4 weeks or 1 month, e.g., 1 week (QW), 2 weeks (Q2W), or 4 weeks (Q4W). In a particular embodiment, the maintenance administration is administration of a dose of 6 mg/kg at 4-week intervals (Q4W), which can be referred to as every-4-week 6 mg/kg antibody maintenance dosing regimen. In another embodiment, the maintenance administration is administration of an antibody dose of 3 mg/kg, 3.5 mg/kg, 3.6 mg/kg, 4 mg/kg, 4.2 mg/kg, 4.5 mg/kg, 4.8 mg/kg, 5 mg/kg, 5.4 mg/kg, 5.5 mg/kg, or 6 mg/kg at 2-week intervals (Q2W). In yet another embodiment, the maintenance administration is administration of an antibody dose of 1.5 mg/kg, 1.75 mg/kg, 1.8 mg/kg, 2 mg/kg, 2.1 mg/kg, 2.25 mg/kg, 2.4 mg/kg, 2.5 mg/kg, 2.7 mg/kg, 2.75 mg/kg, or 3 mg/kg at 1-week intervals (QW).

In another aspect, a different or alternative dose and administration interval can be applicable.

In certain embodiments, a different or alternative dose and administration interval can be applicable after the above-mentioned initial doses and administration intervals. More specifically, one may modify the above-mentioned every-4-week 6 mg/kg antibody maintenance dosing regimen to apply a different, alternative, or modified dose and administration interval. There is no particular limitation on the number of times that one can change the maintenance dose and administration interval. The changes of the maintenance dose and administration interval may be made several times, e.g., once to four times. In other words, one to several, e.g. one to five, different doses and administration intervals may be applied in a sequential manner, such as (0) administering a certain dose at a certain administration interval, (1) stopping administering that dose at that administration interval and starting administering a first modified dose at a first modified administration interval, (2) stopping administering the first modified dose at the first modified administration interval and starting administering a second modified dose at a second modified administration interval, (3) stopping administering the second modified dose at the second modified administration interval and starting administering a third modified dose at a third modified administration interval, and (4) stopping administering the third modified dose at the third modified administration interval and starting administering a fourth modified dose at a fourth modified administration interval.

In some embodiments, the modified dose may be applied from the beginning, without using the above-mentioned every-4-week 6 mg/kg antibody maintenance dosing regimen.

The number of times the dose is administered in maintenance administration is not particularly limited, and the number is for example at least once, at least twice, at least three times, at least four times, at least five times, at least six times, at least seven times, at least eight times, at least nine times, at least ten times, at least 15 times, at least 20 times, at least 25 times, at least 35 times, at least 40 times, at least 50 times, at least 60 times, at least 70 times, at least 80 times, at least 90 times, at least 100 times, at least 500 times, at least 1000 times, at least 10000 times, or more.

In a particular embodiment, the antibody (emicizumab) is administered as follows:
(1) subcutaneously administered at an antibody dose of 6 mg/kg once on Day 1;
(2) subcutaneously administered at an antibody dose of 3 mg/kg, 4.5 mg/kg, or 6 mg/kg once on Day 2; and
(3) subcutaneously administered repeatedly from Day 8, at an antibody dose of 1.5 mg/kg once every week, at an antibody dose of 3 mg/kg once every 2 weeks, or at an antibody dose of 6 mg/kg once every 4 weeks.

In another embodiment, the antibody (emicizumab) is administered as follows:
(1) subcutaneously administered at an antibody dose of 6 mg/kg once on Day 1;
(2) subcutaneously administered at an antibody dose of 6 mg/kg once on Day 2;
(3) subcutaneously administered at an antibody dose of 1.5 mg/kg, 3 mg/kg, 4.5 mg/kg, or 6 mg/kg once on Day 3; and
(4) subcutaneously administered repeatedly from Day 8, at an antibody dose of 1.5 mg/kg once a week, at an antibody dose of 3 mg/kg once every 2 weeks, or at an antibody dose of 6 mg/kg once every 4 weeks.

In yet another embodiment, the antibody (emicizumab) is administered as follows:
(1) subcutaneously administered at an antibody dose of 6 mg/kg once on Day 1; and
(2) subcutaneously administered at an antibody dose of 4.5 mg/kg once on Day 2, and subcutaneously administered repeatedly from Day 8, at an antibody dose of 1.75 mg/kg once a week, or at an antibody dose of 3.5 mg/kg once every 2 weeks;
   subcutaneously administered at an antibody dose of 4.8 mg/kg once on Day 2, and
   subcutaneously administered repeatedly from Day 8, at an antibody dose of 1.8 mg/kg once a week, or at an antibody dose of 3.6 mg/kg once every 2 weeks;
   subcutaneously administered at an antibody dose of 6 mg/kg once on Day 2, and subcutaneously administered repeatedly from Day 8, at an antibody dose of 2 mg/kg once a week, or at an antibody dose of 4 mg/kg once every 2 weeks; or
   subcutaneously administered at an antibody dose of 3.3 mg/kg once on Day 2, subcutaneously administered at an antibody dose of 3.3 mg/kg once on Day 3, and subcutaneously administered repeatedly from Day 8, at an antibody dose of 2.1 mg/kg once a week, or at an antibody dose of 4.2 mg/kg once every 2 weeks.

In yet another embodiment, the antibody (emicizumab) is administered as follows:
(1) subcutaneously administered at an antibody dose of 6 mg/kg once on Day 1;
(2) subcutaneously administered at an antibody dose of 6 mg/kg once on Day 2;
(3) subcutaneously administered at an antibody dose of 1.5 mg/kg once on Day 3, and subcutaneously administered repeatedly from Day 8, at an antibody dose of 2.25 mg/kg once a week, or at an antibody dose of 4.5 mg/kg once every 2 weeks;
   subcutaneously administered at an antibody dose of 2.4 mg/kg once on Day 3, and
   subcutaneously administered repeatedly from Day 8, at an antibody dose of 2.4 mg/kg once a week, or at an antibody dose of 4.8 mg/kg once every 2 weeks;
   subcutaneously administered at an antibody dose of 3 mg/kg once on Day 3, and subcutaneously administered repeatedly from Day 8, at an antibody dose of 2.5 mg/kg once a week, or at an antibody dose of 5 mg/kg once every 2 weeks;
   subcutaneously administered at an antibody dose of 4.2 mg/kg once on Day 3, and
   subcutaneously administered repeatedly from Day 8, at an antibody dose of 2.7 mg/kg once a week, or at an antibody dose of 5.4 mg/kg once every 2 weeks;
   subcutaneously administered at an antibody dose of 4.5 mg/kg once on Day 3, and
   subcutaneously administered repeatedly from Day 8, at an antibody dose of 2.75 mg/kg once a week, or at an antibody dose of 5.5 mg/kg once every 2 weeks; or
   subcutaneously administered at an antibody dose of 6 mg/kg once on Day 3, and subcutaneously administered repeatedly from Day 8, at an antibody dose of 3 mg/kg once a week, or at an antibody dose of 6 mg/kg once every 2 weeks.

### <When maintenance dosing is at 1-week intervals>

| Antibody dose on the first day (Day 1) (mg/kg) | Antibody dose on Day 2 (mg/kg) | Antibody dose on Day 3 (mg/kg) | Antibody dose on Day 8 and later (mg/kg QW) |
|---|---|---|---|
| 6 | 3 | - | 1.5 |
| 6 | 4.5 | - | 1.5 |
| 6 | 6 | - | 1.5 |
| 6 | 6 | 1.5 | 1.5 |
| 6 | 6 | 3 | 1.5 |
| 6 | 6 | 4.5 | 1.5 |
| 6 | 6 | 6 | 1.5 |
| 6 | 4.5 | - | 1.75 |
| 6 | 4.8 | - | 1.8 |
| 6 | 6 | - | 2 |
| 6 | 3.3 | 3.3 | 2. 1 |
| 6 | 6 | 1.5 | 2. 25 |
| 6 | 6 | 2.4 | 2.4 |
| 6 | 6 | 3 | 2.5 |
| 6 | 6 | 4.2 | 2.7 |
| 6 | 6 | 4.5 | 2. 75 |
| 6 | 6 | 6 | 3 |

### <When maintenance dosing is at 2-week intervals>

| Antibody dose on the first day (Day 1) (mg/kg) | Antibody dose on Day 2 (mg/kg) | Antibody dose on Day 3 (mg/kg) | Antibody dose on Day 8 and later (mg/kg Q2W) |
|---|---|---|---|
| 6 | 3 | - | 3 |
| 6 | 4.5 | - | 3 |
| 6 | 6 | - | 3 |
| 6 | 6 | 1.5 | 3 |
| 6 | 6 | 3 | 3 |
| 6 | 6 | 4.5 | 3 |
| 6 | 6 | 6 | 3 |
| 6 | 4.5 | - | 3.5 |
| 6 | 4.8 | - | 3.6 |
| 6 | 6 | - | 4 |
| 6 | 3.3 | 3.3 | 4.2 |
| 6 | 6 | 1.5 | 4.5 |
| 6 | 6 | 2.4 | 4.8 |
| 6 | 6 | 3 | 5 |
| 6 | 6 | 4.2 | 5.4 |
| 6 | 6 | 4.5 | 5.5 |
| 6 | 6 | 6 | 6 |

### <When maintenance dosing is at 4-week intervals>

| Antibody dose on the first day (Day 1) (mg/kg) | Antibody dose on Day 2 (mg/kg) | Antibody dose on Day 3 (mg/kg) | Antibody dose on Day 8 and later (mg/kg Q4W) |
|---|---|---|---|
| 6 | 3 | - | 6 |
| 6 | 4.5 | - | 6 |
| 6 | 6 | - | 6 |
| 6 | 6 | 1.5 | 6 |
| 6 | 6 | 3 | 6 |
| 6 | 6 | 4.5 | 6 |
| 6 | 6 | 6 | 6 |

In some embodiments, regimens of the invention can be applicable for patients who are at risk of bleeding, or excessive bleeding. The regimens of the invention can be applicable in a method for preventing and/or treating bleeding in such patients, or for increasing blood clotting activity in such patients, or for reducing excessive bleeding in such patients. Herein, "preventing" bleeding refers to reducing the incidence of bleeding (reducing the frequency of bleeding episodes) or reducing the likelihood of bleeding in a patient. In certain embodiments, excessive bleeding in such patients is caused by a decrease or deficiency in the activity of FVIII and/or FVIIIa. In a certain embodiment, patents who are at risk of bleeding have hemophilia, which may be hemophilia A or severe hemophilia A.

In some embodiments, regimens of the invention can be applicable for patients with hemophilia A and preferably patients with hemophilia A having FVIII inhibitors and/or patients with hemophilia A, in particular, acquired hemophilia A, not having FVIII inhibitors.

The term "inhibitor patient" as used herein refers to a patient with hemophilia A having FVIII inhibitors.

The term "non-inhibitor patient" as used herein refers to a patient with hemophilia A not having FVIII inhibitors.

In some embodiment, regimens of the invention can be applicable for patients with severe hemophilia A.

In some embodiments, the pharmaceutical compositions according to the administration regimen of the present invention are used under the administration of an immunosuppressive drug. Examples of "immunosuppressive drugs" include steroid drugs such as prednisolone and methylprednisolone, cyclophosphamide, rituximab, and cyclosporin A.
"Used under the administration of an immunosuppressive drug" means that the administration of emicizumab is performed at the same time as the administration of the immunosuppressive drug, in parallel with the administration of the immunosuppressive drug, before the administration of the immunosuppressive drug, or after the administration of the immunosuppressive drug.

Under the administration of an immunosuppressive drug, the effect of the immunosuppressive drug is determined by measuring FVIII activity, inhibitor titer, and/or activated partial thromboplastin time (APTT), etc. When administration of a pharmaceutical composition comprising emicizumab is done in parallel with the administration of an immunosuppressive drug, the effect of the immunosuppressive drug could be determined by measuring FVIII activity or the like, which is performed neutralizing the activity of emicizumab with an anti-emicizumab antibody (neutralizing antibody against emicizumab) or the like, or using a chromogenic substrate assay using blood coagulation factors of an animal species with which emicizumab shows no reactivity or the like. In addition, if the immunosuppressive drug is judged to be therapeutically effective as a result of the effect determination, the administration of emicizumab will be discontinued.

The pharmaceutical compositions according to the administration regimens of the present invention can also be applied to patients who cannot receive immunotherapy due to reasons such as having complications that are undesired in immunotherapy, having hemophilia A resistant to immunotherapy, or exhibiting clinically problematic side effects due to immunotherapy. The pharmaceutical compositions can also be applied to patients who have not been able to obtain a significant therapeutic effect by immunotherapy.

In some embodiments, regimens of the invention can be applicable for adult patients, and/or pediatric patients and/or such special population of patients likely to exhibit lower exposure.

The dosage regimen is determined, for example, by considering the effects and safety. Furthermore, the dosage regimen is determined by considering the convenience of the patient, within the range that does not impair the effectiveness and safety. For example, the dosage regimen for a hemophilia A patient can be determined by considering the effects of preventing bleeding in patients and clinically acceptable safety.

The present invention provides a product comprising at least (i) a container; (ii) a pharmaceutical composition in that container, which comprises emicizumab; and (iii) a document instructing administration of emicizumab according to any one of the dosing regimens described above. In addition, a label, syringe, syringe needle, pharmaceutically acceptable medium, alcohol-soaked cotton, adhesive bandage, and such may be packaged in the product. The container is, for example, a bottle, a glass bottle, or a syringe, and can be produced from various materials such as glass or plastic. Administration-supporting devices may be attached to the product. A pharmaceutical composition is stored in the container, and the mouth of the container is sealed with a rubber stopper or such. For example, a label indicating that the pharmaceutical composition is to be used for preventing or treating selected pathological conditions is attached to the container. The document of (iii) may include instructions that specify the doses, administration frequency or intervals for loading administration and maintenance administration, according to the dosing regimens as described above. Examples of the document include appended documents, package inserts, and prescribing information, attached to medical drugs.

Treatment of hemophilia refers to, for example, stopping bleeding by administering the composition to a hemophilia patient who is actually showing bleeding symptoms (treatment of bleeding) and/or reducing the bleeding frequency by administering the composition to a patient who had shown bleeding to prevent manifestation of bleeding symptoms in advance (prevention of bleeding), but it is not limited thereto. Treatment and prevention of bleeding may be understood as having the same meaning in certain cases and such treatment and prevention of bleeding may be called prophylaxis therapy or regular administration therapy of emicizumab.

Prevention of hemophilia refers to, for example, reducing the incidence of hemophilia or reducing the likelihood of hemophilia.

Herein, bleeding that is examined and counted as the number of bleeding events in a patient is, for example, bleeding that required hemostatic treatment by coagulation factor formulations. Coagulation factor formulations refer to, for example, FVIII formulations and bypassing agents (activated prothrombin complex formulations, recombinant FVIIa formulations, and such).

The number of bleedings per year (the Annualized Bleeding Rate (ABR)) is calculated as, for example: (number of bleeding events x 365.25) / number of days of observation.

All prior art references cited herein are incorporated by reference into this description.

### [Examples]

The present invention is specifically illustrated below with reference to Examples, but it is not construed as being limited thereto.

In the Examples below, data obtained in emicizumab clinical trials in patients with congenital hemophilia A was used to construct an emicizumab population pharmacokinetic model to determine the dosage and administration of emicizumab treatment for patients with acquired hemophilia A. The approved dosages and administrations of emicizumab for congenital hemophilia A, which comprise repeated subcutaneous administration of a dose of 3 mg/kg for 4 weeks at weekly intervals (loading) followed by a dose of 1.5 mg/kg at weekly intervals, 3 mg/kg at 2-week intervals, or 6 mg/kg at 4-week intervals thereafter (maintenance), will be referred to as approved 1-week, 2-week, or 4-week regimens, respectively.

### [Reference Example 1] Superiority to bypassing agents in patients with congenital hemophilia A

The superiority of the approved emicizumab 1-week regimen for its bleeding-preventing effect over bypassing agents, which are used as the standard hemostatic treatment in acquired hemophilia A, has been shown in patients with congenital hemophilia A with inhibitors through the emicizumab clinical development program for congenital hemophilia A.

In a global phase III clinical trial (Study BH29884) in adult/adolescent patients with congenital hemophilia A with inhibitors, when emicizumab was regularly administered at the approved 1-week regimen to patients who had received episodic hemostatic therapy with bypassing agents prior to study participation (A group), there was a statistically-significant and clinically-meaningful reduction in the annualized bleeding rate of treatment-requiring bleeding as compared to the group of no regular emicizumab administration (B_{control} group). In addition, in the same study, when emicizumab was regularly administered at the same dosage and administration regimen to patients who, before participating in the study, had participated in a non-interventional study (Study BH29768) and received episodic hemostatic therapy or regular infusion with bypassing agents (A_{NIS} group or C_{NIS} group, respectively), a decrease in the annualized bleeding rate of treatment-requiring bleeding was observed in both groups as compared to while receiving episodic hemostatic therapy or regular infusion with bypassing agents. Also in a global phase III clinical trial (Study BH29992) in pediatric patients with congenital hemophilia A with inhibitors, when emicizumab was regularly administered at the same dosage and administration regimen to patients who, before participating in the study, had participated in Study BH29768 and received episodic hemostatic therapy or regular infusion with bypassing agents, a decrease in the annualized bleeding rate of treatment-requiring bleeding was observed as compared to while receiving episodic hemostatic therapy or regular infusion with bypassing agents.

### [Reference Example 2] Exposure-efficacy relationship in patients with congenital hemophilia A

The superiority of the approved emicizumab 1-week regimen over bypassing agents shown in patients with congenital hemophilia A with inhibitors is considered able to be generalized among the dosages and administrations by which plasma emicizumab concentrations exceed 30 µg/mL in most patients.

Throughout Study BH29884, Study BH29992, a global phase III clinical trial in adult/adolescent patients with congenital hemophilia A without inhibitors (Study BH30071), and a global phase III clinical trial in adult/adolescent patients with congenital hemophilia A with or without inhibitors (Study BO39182), the bleeding-preventing effect of regular emicizumab administration at the approved 1-week, 2-week, or 4-week interval regimen was comparable regardless of the presence or absence of FVIII inhibitors and the dosage and administration. When the approved 1-week, 2-week, or 4-week interval regimen was administered, no obvious association was found between the average plasma concentration of emicizumab over the entire efficacy analysis period and the annualized bleeding rate based on treatment-requiring bleeding, suggesting that a maximum or near maximum bleeding-preventing effect of emicizumab was obtained in most patients regardless of the dosage and administration. In addition, a population exposure-efficacy analysis predicted that the effect of reducing annualized bleeding rate based on treatment-requiring bleeding would reach a maximum in the presence of emicizumab at plasma concentrations of above approximately 30 µg/mL, and it was considered that the differences in the time course of plasma emicizumab concentration among the approved 1-week, 2-week, and 4-week interval regimens, where plasma emicizumab concentrations are estimated to exceed 30 µg/mL in most patients, have no effect on the efficacy.

### [Example 1] Optimal dosage and administration for patients with acquired hemophilia A

### (1) Estimated effective concentration in patients with acquired hemophilia A

Since the molecular structure of emicizumab is different from FVIII, it is considered that FVIII inhibitors do not affect the FVIII function-substituting activity of emicizumab, and that comparable bleeding-preventing effect can be obtained by regular administration of emicizumab regardless of the presence or absence of FVIII inhibitors. In patients with congenital hemophilia A, the FVIII function-substituting activity and bleeding-preventing effect of emicizumab were similar between patients with and without inhibitors.

As a pharmacological study (*in vivo*) for supporting the efficacy of emicizumab, bleeding-preventing and hemostatic effects of emicizumab were suggested in a model in which bleeding is induced by intramuscular puncture, etc. after administering an anti-FVIII antibody to cause an acquired hemophilia A state in cynomolgus monkeys (cynomolgus monkey FVIII-neutralized hemophilia A/puncture bleeding model), and in a model in which bleeding is induced by daily actions and operations under the condition where FVIII activity is decreased by the same method (cynomolgus monkey FVIII-neutralized hemophilia A/spontaneous bleeding model). In addition, it has been reported that, as a result of performing a comprehensive coagulability test using plasma samples obtained from patients with acquired hemophilia A spiked with multiple concentrations of emicizumab *ex vivo,* an improvement in coagulability was observed in a manner dependent on the plasma concentration of emicizumab (Blood 126 (23), 3565 (2015)).

From these results, it is expected that regular administration of emicizumab will have a bleeding-preventing effect even in patients with acquired hemophilia A, and will show a similar exposure-efficacy relationship to in patients with congenital hemophilia A. Therefore, it was decided to set the estimated effective concentration in patients with acquired hemophilia A as 30 µg/mL, which is considered to enable generalization of the superiority to bypassing agents, the standard hemostatic drugs used in acquired hemophilia A.

### (2) Estimated optimal dosage and administration for patients with acquired hemophilia A

Unlike congenital hemophilia A, which is caused by a congenital deficiency or dysfunction of FVIII and requires lifelong treatment for hemostasis and bleeding prevention, the period of exposure to bleeding risk due to acquired hemophilia A is considered to be limited to the period during which FVIII activity is reduced due to the presence of FVIII inhibitors. As an indicator of the time required to restore FVIII activity and to minimize the risk of bleeding by immunosuppressive therapy (IST), there is a report that the median time between initiation of IST and achievement of partial remission (PR) is 31 days (Blood 125 (7), 1091-1097 (2015)). The length of this period corresponds to the period of loading administration for the approved dosage and administration regimens of emicizumab for congenital hemophilia A (4 weeks), and it is also the period during which plasma emicizumab concentration trough level reaches a steady state at the approved 1-week regimen. Therefore, if it is assumed that the regular administration of emicizumab is started at the same time as the start of IST as an example of a future treatment scheme for acquired hemophilia A, it is expected that the approved dosage and administration regimen would lead to a consequence that, although plasma emicizumab concentration is still on the increase before the achievement of PR when the bleeding risk is high, approximately half of the patients have already achieved PR and do not require any more emicizumab administration after 4 weeks of the start of administration (Day 29) when the plasma emicizumab concentration finishes increasing. Therefore, the approved dosage and administration regimens may not maximize the potential of regular emicizumab administrations to prevent bleeding in acquired hemophilia A.

Based on these assumptions, the dosage and administration of emicizumab suitable for the treatment scheme and clinical course of acquired hemophilia A was examined. A population pharmacokinetic model constructed using plasma emicizumab concentration data in patients with congenital hemophilia A was used to simulate time courses of in plasma emicizumab concentration in patients with acquired hemophilia A. A one-compartment model with a first-order absorption process and a first-order elimination process was selected as the structural model of the population pharmacokinetic model. An exponential error model and a mixed error model were selected as the inter-individual variability model and the residual variability model, respectively. Table 1 shows the parameter estimates of the population pharmacokinetic model, and Table 2 shows the effects of the included covariates. The distribution (mean ± standard deviation) or breakdown of the covariates in the simulation was: 74.9 ± 10.5 years for age, 69 ± 13.3 kg for body weight, 45.0 ± 4.13 g/L for albumin, and non-Black/non-African American for race. Bioavailability in patients aged more than 77 years was assumed to be consistently equivalent to that in patients aged 77 years. The analysis was performed using NONMEM version 7.2.0 or 7.4.3 (ICON Development Solutions, Ellicott City, MD, USA).

**[Table 1]**

| Parameter Estimates of the Population Pharmacokinetic Model | | | | | |
|---|---|---|---|---|---|
| Parameter | Unit | Estimate | RSE (%) | 95% CI (lower, upper) | Shrinkage (%) |
| Fixed Effects | | | | | |
| CL/F | L/day | 0.272 | 1.9 | (0.262, 0.282) | |
| V/F | L | 10.4 | 1.9 | (10.0, 10.8) | |
| KA | 1/day | 0.536 | 7.1 | (0.462, 0.610) | |
| Random Effects BPV | | | | | |
| CL/F | CV% | 28.7 | 8.6^{a} | | 3.7 |
| V/F | CV% | 25.9 | 8.9^{a} | | 10.3 |
| KA | CV% | 72.5 | 14.7^{a} | | 40.6 |
| Correlation CL/F-V/F | - | 0.217 | 31.8^{b} | | |
| Correlation CL/F-KA | - | -0.341 | 25.0^{b} | | |
| Covariate Effects | | | | | |
| Effect of BW on CL/F | - | 0.911 | 3.2 | (0.854, 0.968) | |
| Effect of ALB on CL/F | - | 1.57 | 28.4 | (0.696, 2.44) | |
| Effect of BW on V/F | - | 1.00 | 3.0 | (0.941, 1.06) | |
| Effect of BLACK on V/F | - | -0.215 | 19.7 | (-0.298, -0.132) | |
| Effect of AGE > 30 years | - | 6.51 × 10⁻¹ | 16.3 | (4.43 × 10¹, 8.59 × 10⁻¹) | |
| on Fᵣₑₗ | | | | | |
| Error Model | | | | | |
| σ₁ (additive) | µg/mL | 0.025 fixed | - | | |
| σ₂ (proportional) | % | 14.6 | 2.0 | (14.0, 15.2) | |

| | | | | | |
|---|---|---|---|---|---|
| AGE = age; ALB = albumin; BLACK = Black/African American; BPV = between patient variability; BW = body weight; CI = confidence interval; CL/F = apparent total clearance; CV = coefficient of variation; Fᵣₑₗ = relative bioavailability; KA = absorption rate constant; RSE = relative standard error; σ = residual variability; V/F = apparent volume of distribution. ^{a} RSE calculated for variance. ^{b} RSE calculated for covariance. | | | | | |

**[Table 2]**

| Effects of the Covariates Included in the Population Pharmacokinetic Model | | | |
|---|---|---|---|
| Statistically Significant Effect | Relationship | Covariate Range [min, max] | % Change in PK Parameters from Typical Value |
| BW (kg) on CL/F | CL/F = 0.272 × (BW / 70)^{0.911} | [9, 156] | [-85, +108] |
| ALB (g/L) on CL/F | CL/F = 0.272 × (1 - 0.0157 × (ALB - 45)) | [33, 57] | [+19, -19] |
| BW (kg) on V/F | V/F = 10.4 × (BW / 70)^{1.00} | [9, 156] | [-87, +123] |
| BLACK on V/F | V/F = 10.4 × (1 - 0.215 × BLACK) | Non Black / Black | 0 / -22 |
| AGE (y) on Fᵣₑₗ | If AGE ≤ 30; Fᵣₑₗ = 1 | [1.22, 30] | [0, 0] |
| | If AGE > 30; Fᵣₑₗ = 1 - 0.00651 × (AGE - 30) | [30, 77] | [0, -31] |

| | | | |
|---|---|---|---|
| AGE = age; ALB = albumin; BLACK = Black/African American; BW = body weight; CL/F = apparent total clearance; Fᵣₑₗ = relative bioavailability; PK = pharmacokinetic(s); V/F = apparent volume of distribution. | | | |

The dosage and administration regimens considered included, in addition to the approved 1-week regimen, a dosage and administration regimen in which 6 mg/kg and 3 mg/kg are subcutaneously administered on Day 1 and Day 2, respectively (loading administration), and 1.5 mg/kg is subcutaneously administered repeatedly from Day 8 at weekly intervals (maintenance administration) (daily loading + 1-week interval maintenance administration regimen), which regimen is expected to allow the plasma emicizumab concentration to reach the estimated effective concentration of 30 µg/mL at an earlier stage and to stabilize at an earlier stage. The dose of 6 mg/kg given on Day 1 is the highest dose tested through the clinical development program for congenital hemophilia A and is the highest approved dosage per administration.

Simulations of the time courses of plasma emicizumab concentration when the approved 1-week interval regimen and the presently disclosed daily loading + 1-week interval maintenance administration regimen are given to patients with acquired hemophilia A are shown in Figs. 1 and 2, respectively. With the approved 1-week regimen, the median plasma emicizumab concentration trough levels after 1 week (Day 8) and 4 weeks (Day 29) after the start of administration are predicted to be 11.6 and 37.8 µg/mL, respectively. On the other hand, with the daily loading + 1-week interval maintenance administration regimen of the present disclosure, the median plasma emicizumab concentration trough levels after 1 week (Day 8) and 4 weeks (Day 29) after the start of administration are predicted to be 34.6 and 36.9 µg / mL, respectively. With the daily loading + 1 week interval maintenance administration regimen of the present disclosure, since it is predicted that the plasma emicizumab concentration will exceed 30 µg/mL in most patients by 1 week after the start of administration (Day 8), and that the plasma emicizumab concentration trough level will reach a steady state by 2 weeks after the start of administration (Day 15), the effect of regular emicizumab administration for the purpose of preventing bleeding in acquired hemophilia A may be obtained promptly and stably.

### (3) Safety margins for the estimated optimal dosage and administration

As a toxicity evaluation program for emicizumab, 13-week and 26-week subcutaneous administration studies and a 4-week intravenous administration study using cynomolgus monkeys were conducted. The no observed adverse effect levels (NOAELs) were determined to be weekly doses of 30, 30 and 100 mg/kg, respectively, and the mean maximum plasma concentrations at the final dose of NOAEL (mean initial plasma concentrations in the intravenous administration study) were 1070 to 1200, 1340 to 1370, and 3550 to 3560 µg/mL, respectively. The estimated plasma emicizumab concentration in patients with acquired hemophilia A at the daily loading + 1-week interval maintenance administration regimen of the present disclosure (Fig. 2) is considered to be sufficiently higher than the exposure levels at these NOAELs in cynomolgus monkeys. In humans, the estimated exposure level at the daily loading + 1-week interval maintenance administration regimen of the present disclosure (Fig. 2) is below the mean steady-state plasma concentration trough level (120 µg/mL) for weekly 3 mg/kg administration, which is the highest dose for which the tolerability has been confirmed through the clinical development program for congenital hemophilia A.

The administration interval of loading administration for the daily loading + 1-week interval maintenance administration regimen of the present disclosure, which is 1 day, is a short administration interval that has not been experienced in previous non-clinical and clinical studies. However, based on the toxicity study results of intravenous administration in which the plasma emicizumab concentration rapidly increases immediately after administration, it is considered that the safety margin for acute toxicity is ensured. The mean initial plasma concentration at the first dose of NOAEL was 2160 to 2270 µg/mL in the 4-week intravenous administration study using cynomolgus monkeys, and it is considered that this sufficiently covers the plasma emicizumab concentration during loading administration at the daily loading + 1-week interval maintenance administration regimen of the present disclosure (Fig. 2).

These results suggest that the safety margin is ensured for the daily loading + 1-week interval maintenance administration regimen of the present disclosure.

### [Example 2]

A multicenter, open-label, non-randomized, phase III clinical trial (hereinbelow, "this clinical trial") is conducted to investigate the safety, efficacy, pharmacokinetics and pharmacodynamics of emicizumab subcutaneously administered to patients with acquired hemophilia A at the daily loading + 1-week interval maintenance administration regimen of the present disclosure.

In this clinical trial, emicizumab is subcutaneously administered at 6 mg/kg and 3 mg/kg on Day 1 and Day 2, respectively (loading administration), and 1.5 mg/kg is subcutaneously administered repeatedly from Day 8 at weekly intervals (maintenance administration). Subjects enrolled in this study will continue to receive emicizumab until they meet the criteria for completion/discontinuation of emicizumab administration or discontinue the study. Subjects who have completed/discontinued emicizumab administration will be followed up for safety for 24 weeks after the completion/discontinuation of emicizumab administration. The criteria for completion of emicizumab administration comprise: FVIII activity (non-responsive to emicizumab; one-stage clotting assay with emicizumab neutralization) has exceeded 50 IU/dL and more than 72 hours have passed since the last administration of blood coagulation factor products for the latest treatment-requiring bleeding. The criteria for discontinuation of emicizumab administration comprise pregnancy and occurrence of unacceptable adverse events.

This study consists of two cohorts, Cohort 1 in which emicizumab is administered with immunosuppressive therapy (under immunosuppressive drug administration) and Cohort 2 in which emicizumab is administered without immunosuppressive therapy. First, a minimum of 10 patients with acquired hemophilia A aged 18 years or older who are scheduled to immediately undergo or are undergoing immunosuppressive therapy at the time of study enrollment are enrolled in Cohort 1.

In order to confirm the optimal dosage and administration of emicizumab for patients with acquired hemophilia A, the appropriateness of the dosage and administration in patients with acquired hemophilia A will be evaluated by an interim data review. When the first 6 patients enrolled in Cohort 1 reach 4 weeks after the start of emicizumab administration, or earlier if necessary, the sponsor will comprehensively evaluate the safety, efficacy, pharmacokinetics and pharmacodynamics up until that time point in consultation with the medical expert, and determine the appropriateness of the dosage and administration. If it is determined that the dosage and administration need to be adapted, it may be necessary to enroll additional patients in Cohort 1 and evaluate new dosage and administration.

After the dosage and administration are determined to be appropriate, enrollment of patients in Cohort 2 is started-at least one patient aged 18 years or older with acquired hemophilia A, for whom the principal investigator (sub-investigator) determines that it is difficult to perform immunosuppressive therapy at the time of study enrollment, is enrolled.

The primary analysis will be performed when all of the following conditions are met. If it is determined that the dosage and administration need to be adapted, the analysis will be conducted when all of the following conditions are met in the subjects who started the clinical trial with the adapted dosage and administration.
- At least 10 subjects have been enrolled in Cohort 1.
- Three or more subjects in Cohort 1 have met the criteria for completing emicizumab administration and then completed the safety follow-up (24 weeks after completion of emicizumab administration) or discontinued the clinical trial during the safety follow-up period.
- All the subjects in Cohort 1 have reached either completion/discontinuation of emicizumab administration, continuation of emicizumab administration for 24 weeks or longer, or discontinuation of the clinical trial.

Safety is evaluated based on adverse events, physical examination findings, vital signs, 12-lead electrocardiogram, laboratory test values, blood coagulation factor VIII (FVIII) inhibitors (non-responsive to emicizumab; one-stage clotting Bethesda assay with emicizumab neutralization), anti-emicizumab antibodies, etc. Efficacy is evaluated based on the number of bleeding episodes requiring treatment with blood coagulation factor products, the usage of blood coagulation factor products, the usage of blood transfusions, changes in hemoglobin levels, etc. Pharmacokinetics is evaluated based on plasma emicizumab concentration. Pharmacodynamics is evaluated based on FVIII activity (non-responsive to emicizumab; one-stage clotting assay with emicizumab neutralization), FVIII activity (non-responsive to emicizumab; chromogenic substrate assay using bovine coagulation factors), FVIII activity (responsive to emicizumab; chromogenic substrate assay using human coagulation factors), activated partial thromboplastin time (APTT), etc.

From the results of this study, it is possible to confirm the utility of emicizumab in patients with acquired hemophilia A at the daily loading + 1-week interval maintenance administration regimen of the present disclosure.

### [Industrial applicability]

The present invention provides administration regimens of pharmaceutical compositions comprising emicizumab that have the potential to effectively prevent and/or treat acquired hemophilia A.

## Claims

1. A pharmaceutical composition for use in treating acquired hemophilia A and/or reducing the incidence of acquired hemophilia A, wherein the composition comprises emicizumab, wherein emicizumab is administered at an antibody dose of 6 mg/kg on Day 1, at an antibody dose of 3 to 6 mg/kg on Day 2, and at an antibody dose of 1.5 to 3 mg/kg weekly from Day 8, wherein the weekly administration from Day 8 is repeated one or more times.

2. The pharmaceutical composition of claim 1, wherein the administration on Day 1, the administration on Day 2, and the weekly administration from Day 8 are each performed in a single dose.

3. The pharmaceutical composition of claim 1 or 2, wherein the administration on Day 1, the administration on Day 2, and the weekly administration from Day 8 are each performed subcutaneously.

4. The pharmaceutical composition of any one of claims 1 to 3, wherein emicizumab is administered at an antibody dose of 3 mg/kg on Day 2.

5. The pharmaceutical composition of any one of claims 1 to 4, wherein emicizumab is administered at an antibody dose of 1.5 mg/kg weekly from Day 8, wherein the weekly administration from Day 8 is repeated one or more times.

6. The pharmaceutical composition of any one of claims 1 to 5, which is used under administration of an immunosuppressive drug.

7. The pharmaceutical composition of claim 6, wherein the immunosuppressive drug is a steroid drug.

8. A product comprising (i) a container; (ii) a pharmaceutical composition comprising emicizumab in the container, and (iii) a document instructing that emicizumab be administered at an antibody dose of 6 mg/kg on Day 1, at an antibody dose of 3 to 6 mg/kg on Day 2, and at an antibody dose of 1.5 to 3 mg/kg weekly from Day 8, and that the weekly administration from Day 8 be repeated one or more times.

9. The product of claim 8, wherein the administration on Day 1, administration on Day 2, and weekly administration from Day 8 are each performed in a single dose.

10. The product of claim 8 or 9, wherein the administration on Day 1, administration on Day 2, and weekly administration from Day 8 are each performed subcutaneously.

11. The product of any one of claims 8 to 10, wherein emicizumab is administered at an antibody dose of 3 mg/kg on Day 2.

12. The product of any one of claims 8 to 11, wherein emicizumab is administered at an antibody dose of 1.5 mg/kg weekly from Day 8, wherein the weekly administration from Day 8 is repeated one or more times.

13. The product of any one of claims 8 to 12, which comprises said document instructing that emicizumab be used in combination with an immunosuppressive drug.

14. A pharmaceutical composition for use in treating acquired hemophilia A and/or reducing the incidence of acquired hemophilia A, which comprises emicizumab, wherein emicizumab is administered at an antibody dose of 6 mg/kg on Day 1, at an antibody dose of 3 to 6 mg/kg on Day 2, and at an antibody dose of 3 to 4 mg/kg every 2 weeks from Day 8, or at an antibody dose of 6 mg/kg every 4 weeks from Day 8, wherein the administration every 2 weeks or every 4 weeks from Day 8 is repeated one or more times.

15. A pharmaceutical composition for use in treating acquired hemophilia A and/or reducing the incidence of acquired hemophilia A, which comprises emicizumab, wherein emicizumab is administered at an antibody dose of 6 mg/kg on Day 1, at an antibody dose of 3 to 6 mg/kg on Day 2, at an antibody dose of 1.5 to 3 mg/kg on Day 8, and at an antibody dose of 3 to 4 mg/kg every 2 weeks from Day 15, or at an antibody dose of 6 mg/kg every 4 weeks from Day 15, wherein the administration every 2 weeks or every 4 weeks from Day 15 is repeated one or more times.


SEQUENCE LISTING

<110>  CHUGAI SEIYAKU KABUSHIKI KAISHA

<120>  PHARMACEUTICAL COMPOSITION FOR USE IN PREVENTION AND/OR TREATMENT
        OF ACQUIRED HAEMOPHILIA A AND ARTICLE OF MANUFACTURE COMPRISING
       SAID PHARMACEUTICAL COMPOSITION

<130>  C1-A1917P

<150>  JP 2019-188099
<151>  2019-10-11

<150>  JP 2020-011992
<151>  2020-01-28

<160>  15

<170>  PatentIn version 3.5

<210>  1
<211>  5
<212>  PRT
<213>  Artificial Sequence

<220>
<223>  Heavy chain variable region CDR1

<400>  1

Tyr Tyr Asp Ile Gln
1               5

<210>  2
<211>  17
<212>  PRT
<213>  Artificial Sequence

<220>
<223>  Heavy chain variable region CDR2

<400>  2

Ser Ile Ser Pro Ser Gly Gln Ser Thr Tyr Tyr Arg Arg Glu Val Lys
1               5                   10                  15

Gly

<210>  3
<211>  14
<212>  PRT
<213>  Artificial Sequence

<220>
<223>  Heavy chain variable region CDR3

<400>  3

Arg Thr Gly Arg Glu Tyr Gly Gly Gly Trp Tyr Phe Asp Tyr

1               5                   10


<210>  4
<211>  123
<212>  PRT
<213>  Artificial Sequence

<220>
<223>  Heavy chain variable region

<400>  4

Gln Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Tyr Tyr
            20                  25                  30


Asp Ile Gln Trp Val Arg Gln Ala Pro Gly Lys Gly Leu Glu Trp Val
        35                  40                  45


Ser Ser Ile Ser Pro Ser Gly Gln Ser Thr Tyr Tyr Arg Arg Glu Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Arg Thr Gly Arg Glu Tyr Gly Gly Gly Trp Tyr Phe Asp Tyr
            100                 105                 110


Trp Gly Gln Gly Thr Leu Val Thr Val Ser Ser
        115                 120


<210>  5
<211>  448
<212>  PRT
<213>  Artificial Sequence

<220>
<223>  Heavy chain

<400>  5

Gln Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Tyr Tyr
            20                  25                  30

Asp Ile Gln Trp Val Arg Gln Ala Pro Gly Lys Gly Leu Glu Trp Val
        35                  40                  45

Ser Ser Ile Ser Pro Ser Gly Gln Ser Thr Tyr Tyr Arg Arg Glu Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Arg Thr Gly Arg Glu Tyr Gly Gly Gly Trp Tyr Phe Asp Tyr
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Cys Ser Arg Ser Thr Ser Glu Ser
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Thr Cys Asn Val
        195                 200                 205

Asp His Lys Pro Ser Asn Thr Lys Val Asp Lys Arg Val Glu Ser Lys
    210                 215                 220

Tyr Gly Pro Pro Cys Pro Pro Cys Pro Ala Pro Glu Phe Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser Gln Glu
            260                 265                 270

Asp Pro Glu Val Gln Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Gly Leu Pro Ser Ser Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Gln Lys Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Glu Gly Asn Val Phe Ser Cys Ser Val Met His
            420                 425                 430

Glu Ala Leu His Asn Arg Tyr Thr Gln Lys Ser Leu Ser Leu Ser Pro
        435                 440                 445

<210>  6
<211>  5
<212>  PRT
<213>  Artificial Sequence

<220>
<223>  Heavy chain variable region CDR1

<400>  6

Asp Asn Asn Met Asp
1               5

<210>  7
<211>  17
<212>  PRT
<213>  Artificial Sequence

<220>
<223>  Heavy chain variable region CDR2

<400>  7

Asp Ile Asn Thr Arg Ser Gly Gly Ser Ile Tyr Asn Glu Glu Phe Gln
1               5                   10                  15

Asp

<210>  8
<211>  10
<212>  PRT
<213>  Artificial Sequence

<220>
<223>  Heavy chain variable region CDR3

<400>  8

Arg Lys Ser Tyr Gly Tyr Tyr Leu Asp Glu
1               5                   10

<210>  9
<211>  119
<212>  PRT
<213>  Artificial Sequence

<220>
<223>  Heavy chain variable region

<400>  9

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asp Asn
            20                  25                  30

Asn Met Asp Trp Val Arg Gln Ala Pro Gly Gln Gly Leu Glu Trp Met
        35                  40                  45

Gly Asp Ile Asn Thr Arg Ser Gly Gly Ser Ile Tyr Asn Glu Glu Phe
    50                  55                  60

Gln Asp Arg Val Ile Met Thr Val Asp Lys Ser Thr Asp Thr Ala Tyr
65                  70                  75                  80

Met Glu Leu Ser Ser Leu Arg Ser Glu Asp Thr Ala Thr Tyr His Cys
                85                  90                  95

Ala Arg Arg Lys Ser Tyr Gly Tyr Tyr Leu Asp Glu Trp Gly Glu Gly
            100                 105                 110

Thr Leu Val Thr Val Ser Ser

        115

<210>  10
<211>  444
<212>  PRT
<213>  Artificial Sequence

<220>
<223>  Heavy chain

<400>  10

Gln Val Gln Leu Val Gln Ser Gly Ser Glu Leu Lys Lys Pro Gly Ala
1               5                   10                  15

Ser Val Lys Val Ser Cys Lys Ala Ser Gly Tyr Thr Phe Thr Asp Asn
            20                  25                  30

Asn Met Asp Trp Val Arg Gln Ala Pro Gly Gln Gly Leu Glu Trp Met
        35                  40                  45

Gly Asp Ile Asn Thr Arg Ser Gly Gly Ser Ile Tyr Asn Glu Glu Phe
    50                  55                  60

Gln Asp Arg Val Ile Met Thr Val Asp Lys Ser Thr Asp Thr Ala Tyr
65                  70                  75                  80

Met Glu Leu Ser Ser Leu Arg Ser Glu Asp Thr Ala Thr Tyr His Cys
                85                  90                  95

Ala Arg Arg Lys Ser Tyr Gly Tyr Tyr Leu Asp Glu Trp Gly Glu Gly
            100                 105                 110

Thr Leu Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Thr Cys Asn Val Asp His Lys Pro
        195                 200                 205

Ser Asn Thr Lys Val Asp Lys Arg Val Glu Ser Lys Tyr Gly Pro Pro
    210                 215                 220

Cys Pro Pro Cys Pro Ala Pro Glu Phe Leu Gly Gly Pro Ser Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Tyr Asn Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Gln Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Glu Gly Asn Val Phe Ser Cys Ser Val Met His Glu Ala Leu His
            420                 425                 430

Asn His Tyr Thr Gln Glu Ser Leu Ser Leu Ser Pro
        435                 440

<210>  11

<211>  11
<212>  PRT
<213>  Artificial Sequence

<220>
<223>  Light chain variable region CDR1

<400>  11

Lys Ala Ser Arg Asn Ile Glu Arg Gln Leu Ala
1               5                   10


<210>  12
<211>  7
<212>  PRT
<213>  Artificial Sequence

<220>
<223>  Light chain variable region CDR2

<400>  12

Gln Ala Ser Arg Lys Glu Ser
1               5


<210>  13
<211>  9
<212>  PRT
<213>  Artificial Sequence

<220>
<223>  Light chain variable region CDR3

<400>  13

Gln Gln Tyr Ser Asp Pro Pro Leu Thr
1               5


<210>  14
<211>  107
<212>  PRT
<213>  Artificial Sequence

<220>
<223>  Light chain variable region

<400>  14

Asp Ile Gln Met Thr Gln Ser Pro Ser Ser Leu Ser Ala Ser Val Gly
1               5                   10                  15


Asp Arg Val Thr Ile Thr Cys Lys Ala Ser Arg Asn Ile Glu Arg Gln
            20                  25                  30


Leu Ala Trp Tyr Gln Gln Lys Pro Gly Gln Ala Pro Glu Leu Leu Ile
        35                  40                  45

Tyr Gln Ala Ser Arg Lys Glu Ser Gly Val Pro Asp Arg Phe Ser Gly
    50                  55                  60

Ser Arg Tyr Gly Thr Asp Phe Thr Leu Thr Ile Ser Ser Leu Gln Pro
65                  70                  75                  80

Glu Asp Ile Ala Thr Tyr Tyr Cys Gln Gln Tyr Ser Asp Pro Pro Leu
                85                  90                  95

Thr Phe Gly Gly Gly Thr Lys Val Glu Ile Lys
            100                 105

<210>  15
<211>  214
<212>  PRT
<213>  Artificial Sequence

<220>
<223>  Light chain

<400>  15

Asp Ile Gln Met Thr Gln Ser Pro Ser Ser Leu Ser Ala Ser Val Gly
1               5                   10                  15

Asp Arg Val Thr Ile Thr Cys Lys Ala Ser Arg Asn Ile Glu Arg Gln
            20                  25                  30

Leu Ala Trp Tyr Gln Gln Lys Pro Gly Gln Ala Pro Glu Leu Leu Ile
        35                  40                  45

Tyr Gln Ala Ser Arg Lys Glu Ser Gly Val Pro Asp Arg Phe Ser Gly
    50                  55                  60

Ser Arg Tyr Gly Thr Asp Phe Thr Leu Thr Ile Ser Ser Leu Gln Pro
65                  70                  75                  80

Glu Asp Ile Ala Thr Tyr Tyr Cys Gln Gln Tyr Ser Asp Pro Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln

145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210